# EUROPÄISCHE PATENTSCHRIFT

(11) **EP 1 119 343 B1**
(45) Veröffentlichungstag und Bekanntmachung des Hinweises auf die Patenterteilung: **05.03.2003**
(21) Anmeldenummer: 99959210.8
(22) Anmeldetag: 08.10.1999
(51) Int. Cl.: A61K 7/42, A61K 7/46

(54) **PARFUMKOMPOSITION MIT LICHTSCHUTZ- UND BRÄUNUNGSEFFEKT**
PERFUME COMPOSITION HAVING A SUN-BLOCK AND TANNING EFFECT
COMPOSITION PARFUMEE AVEC EFFET ANTI-SOLAIRE ET BRONZANT

(30) Priorität: 09.10.1998 DE 19847936
(43) Veröffentlichungstag der Anmeldung: 01.08.2001
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: DE9903298
(87) Internationale Veröffentlichungsnummer: WO00021500

(56) Entgegenhaltungen:
- EP-A- 0 770 381
- WO-A-96/12467
- WO-A-97/25970
- BE-A- 1 002 709
- DE-A- 2 824 198
- DATABASE WPI Week 9523, Derwent Publications Ltd., London, GB; AN 1995-176562, XP002133704 & RU 2 014 824 C1 (PASECHNIK) 30 Juni 1994

## Beschreibung

Die Erfindung betrifft eine Parfümzusammensetzung, die zugleich eine Selbstbräunungswirkung oder eine lichtschützende und selbstbräunende Wirkung aufweist.

Es sind bisher eine Vielzahl von Parfümkompositionen entwickelt worden, die sich im wesentlichen auf unterschiedliche Duftkombinationen oder deren Verkapselung oder Bindung an bestimmte Träger erstrecken. Dazu gehören beispielsweise Parfümpuder, Parfümgele und Parfümsprays. Diese Produkte enthalten keine Lichtschutzmittel mit ausgewiesenem Lichtschutzfaktor oder Selbstbräunungszusätze.

Als Selbstbräunungsmittel ist Dihydroxyaceton bekannt, das als weißes Pulver vorliegt, jedoch bei Kontakt mit einwertigen Alkoholen wie Ethanol zur Bildung von mehr oder weniger eingetrübten Dispersionen neigt, weshalb ein Kontakt mit Ethanol von den Herstellern allgemein als Inkompatibilität ausgewiesen wird.

Aus der EP-A-01812586 sind Selbstbräunungsmittel bekannt, enthaltend Dihydroxyaceton, Ethanol, Polyol, einen organischen Sonnenschutzfilter und 0,3 % Parfüm. In Harry's Cosmetology, 7. Aufl., S. 260 ist eine DHA-haltige Selbstbräunungslotion beschrieben, die neben Ethanol 2 % Parfüm enthält

Der Erfindung liegt die Aufgabe zugrunde, eine Duftkomposition zu entwickeln, die beim Auftragen auf die menschliche Haut eine Brauntönung bewirkt und gegebenenfalls zugleich eine Lichtschutzwirkung gegenüber Haut und Haaren zeigt.

Erfindungsgemäß besteht die Parfümkomposition mit Bräunungseffekt oder Lichtschutz- und Bräunungseffekt aus einer klaren alkohoholisch-wäßrigen Lösung, enthaltend
einen alkohollöslichen organischen Sonnenschutzfilter mit einem Anteil von 0,05 bis 12 Gew-%;
Dihydroxyaceton mit einem Anteil von 0,5 bis 10 Gew-%;
einen oder mehrere einwertige Alkohole mit 2 bis 5 Kohlenstoffatomen mit einem Anteil von 30 bis 85 Gew-%;
einen oder mehrere mehrwertige Alkohole mit 3 bis 5 Kohlenstoffatomen mit einem Anteil von 0,5 bis 15 Gew-%;
Parfümöl mit einem Anteil von 3 bis 20 Gew-%; und
Wasser mit einem Anteil von 3 bis 67,95 Gew-%; und
wobei die Anteile jeweils auf die Gesamtzusammensetzung bezogen sind.

Vorteilhafte Konzentrationen von Dihydroxyaceton liegen im Bereich von 3 bis 8 Gew-%, insbesondere im Bereich von 4 bis 7 Gew-%.

Die Konzentration des alkohollöslichen organischen Sonnenschutzfilters liegt vorteilhaft im Bereich von 1,5 bis 10 Gew-%.

Die Konzentration des Parfümöls liegt vorteilhaft im Bereich von 3 bis 15 Gew-%.

Überraschenderweise bildet sich die sonst mit einem einwertigen Alkohol wie Ethanol auftretende Disharmonie z.B. Trübung mit Dihydroxyaceton nicht, was möglicherweise auf die Anwesenheit des alkohollöslichen organischen Lichtschutzfilters zurückzuführen ist, bisher jedoch noch nicht vollständig geklärt werden konnte.

Als organischer Sonnenschutzfilter wird vorzugsweise Octyl Methoxycinnamate eingesetzt. Andere geeignete Filter sind Benzophenone-3, Butyl Metoxybenzoylmethan oder 4-Methylbenzylidene Camphor.

Der Gehalt an einwertigen Alkoholen liegt vorzugsweise im Bereich von 40 bis 80 Gew-%. Ein besonders geeigneter Alkohol ist Ethanol. Andere mögliche Alkohole sind Isopropanol und n-Propanol.

Der eingesetzte mehrwertige Alkohol kann beispielsweise Glycerin, ein Propandiol oder ein Butandiol sein, vorteilhaft auch ein Gemisch von zwei oder mehreren. Bevorzugt ist beispielsweise ein Gemisch von Glycerin und 1,3-Butandiol, insbesondere in Anteilen von 1:0,5 bis 1:3.

Zusätzlich zu dem alkohollöslichen organischen Sonnenschutzfilter kann ein wasserlöslicher organischer Sonnenschutzfilter enthalten sein. Dazu gehören beispielsweise Benzophenone-3 und Phenylbenzimidazole Sulfonic Acid.

Die Konzentration dieser wasserlöslichen Filter kann im Bereich von 2 bis 15 Gew-% liegen.

Die erfindungsgemäße Parfümkomposition kann mit den entsprechenden Lichtschutzfiltern einen Lichtschutzfaktor von 5 bis 10 haben.

Vorteilhaft liegt die erfindungsgemäße Parfümkomposition als Spray vor. Dies kann sowohl ein Pumpspray oder ein mit Treibgas versehener Spray sein.

Die Erfindung betrifft auch ein Verfahren zur Herstellung einer Parfümkomposition mit Bräunungseffekt oder Lichtschutz- und Bräunungseffekt, wobei dieses Verfahren dadurch gekennzeichnet ist, daß ein Gemisch aus 0,5-15 Gew-% von einem oder mehreren mehrwertigen Alkoholen mit 3 bis 5 Kohlenstoffatomen, 0,5-10 Gew-% Dihydroxyaceton, 0,05-12 Gew-% organischem alkohollöslichem Sonnenschutzfilter und Wasser unter Rühren für einen Zeitraum von 0,5 bis 1,5 Stunden bei 300 bis 600 U/Min hergestellt wird, und in die erhaltene Lösung 30-85 Gew-% von einem oder mehreren einwertigen Alkoholen mit 2 bis 5 Kohlenstoffatomen und 3-13 Gew-% Parfümöl unter Rühren eingetragen werden.

Die erhaltene klare Lösung hat eine ausgezeichnete Stabilität und kann über mehrere Monate gelagert werden.

Die Erfindung soll nachstehend durch Beispiele näher erläutert werden. Alle Angaben erfolgen in Gewichtsprozent, sofern nichts anderes angegeben ist.

| Beispiel 1 **Parfümspray für Körper und Gesicht** | |
|---|---|
| Destilliertes Wasser | 8,0 |
| Glycerin | 4,0 |
| 1,3 Butylen Glycol | 4,0 |
| Dihydroxyaceton | 8,0 |
| Octyl Methoxycinnamate | 2,0 |
| Parfümöl | 5,0 |
| Ethanol | ad 100 |

Es wurde ein Gemisch aus den mehrwertigen Alkoholen, Dihydroxyaceton, Octyl Methoxycinnamate und Wasser unter Rühren bei Raumtemperatur für einen Zeitraum von 70 Minuten bei etwa 500 U/Min hergestellt. In die erhaltene Lösung wurde Ethanol und das Parfümöl ebenfalls unter Rühren eingetragen.

| Beispiel 2 **Parfümspray für Körper und Gesicht mit Licht- schutzfaktor** | |
|---|---|
| Destilliertes Wasser | 10,0 |
| Glycerin | 2,5 |
| 1,3 Butylen Glycol | 3,5 |
| Dihydroxyaceton | 5,0 |
| Octyl Methoxycinnamate | 7,5 |
| Benzophenone-4 | 5,0 |
| Parfümöl | 10,0 |
| Vitamin E | 1,0 |
| Ethanol | ad 100 |

Es wurde wie im Beispiel 1 gearbeitet, wobei Benzophenone-4 und Vitamin E dem zuerst hergestellten Gemisch zugesetzt, und das Ganze bei 520 U/Min für 75 Minuten gerührt wurde. Man erhielt eine Lösung mit einem Lichtschutzfaktor von SPF 10 und zugleich selbstbräunender Wirkung.

| **Beispiel 3 Parfümspray für Haare mit Lichtschutzfaktor** | |
|---|---|
| Destilliertes Wasser | 7,5 |
| Glycerin | 5,0 |
| Dihydroxyaceton | 3,0 |
| Octyl Methoxycinnamate | 7,5 |
| Benzophenone-4 | 5,0 |
| Parfümöl | 13,0 |
| Vitamin E | 1,0 |
| Vitamin B | 1,0 |
| Ethanol | ad 100 |

Es wurde wie im Beispiel 2 gearbeitet. Man erhielt eine schwach selbstbräunende Lösung, die einen Lichtschutzfaktor von SPF 10 hatte. Die Lösung wurde ein einen Sprühbehälter eingebracht.

## Patentansprüche

1. Parfümkomposition mit Bräunungseffekt oder Lichtschutz- und Bräunungseffekt, **dadurch gekennzeichnet, daß** sie in einer klaren alkohoholisch-wäßrigen Lösung
einen alkohollöslichen organischen Sonnenschutzfilter mit einem Anteil von 0,05 bis 12 Gew-%,
Dihydroxyaceton mit einem Anteil von 0,5 bis 10 Gew-%;
einen oder mehrere einwertige Alkohole mit 2 bis 5 Kohlenstoffatomen mit einem Anteil von 30 bis 85 Gew-%;
einen oder mehrere mehrwertige Alkohole mit 3 bis 5 Kohlenstoffatomen mit einem Anteil von 0,5 bis 15 Gew-%;
Parfümöl mit einem Anteil von 3 bis 20 Gew-% ; und
Wasser mit einem Anteil von 3 bis 67,95 Gew-% enthält; und
wobei die Anteile jeweils auf die Gesamtzusammensetzung bezogen sind.

2. Parfümkomposition nach Anspruch 1, **dadurch gekennzeichnet, daß** die Konzentration von Dihydroxyaceton im Bereich von 3 bis 8 Gew-% liegt.

3. Parfümkomposition nach Anspruch 2, **dadurch gekennzeichnet, daß** die Konzentration des Parfümöls im Bereich von 3 bis 15 Gew-% liegt.

4. Parfümkomposition nach Anspruch 1, **dadurch gekennzeichnet, daß** die Konzentration des alkohollöslichen organischen Sonnenschutzfilters im Bereich von 1,5 bis 10 Gew-% liegt.

5. Parfümkomposition nach Anspruch 1, **dadurch gekennzeichnet, daß** der organische Sonnenschutzfilter Octyl Methoxycinnamate ist.

6. Parfümkomposition nach Anspruch 1, **dadurch gekennzeichnet, daß** der Gehalt an einwertigen Alkoholen im Bereich von 40 bis 80 Gew-% liegt.

7. Parfümkomposition nach Anspruch 1, **dadurch gekennzeichnet, daß** der einwertige Alkohol Ethanol ist.

8. Parfümkomposition nach Anspruch 1, **dadurch gekennzeichnet, daß** der mehrwertige Alkohol ein Gemisch von Glycerin und 1,3-Butandiol ist.

9. Parfümkomposition nach einem der Ansprüche 1 bis 8, **dadurch gekennzeichnet, daß** zusätzlich ein wasserlöslicher organischer Sonnenschutzfilter enthalten ist.

10. Parfümkomposition nach einem der Ansprüche 1 bis 9, **dadurch gekennzeichnet, daß** sie einen Lichtschutzfaktor von 5 bis 10 hat.

11. Parfümkomposition nach Anspruch 1, **dadurch gekennzeichnet, daß** sie als Spray vorliegt.

12. Verfahren zur Herstellung einer Parfümkomposition mit Bräunungseffekt oder Lichtschutz- und Bräunungseffekt, **dadurch gekennzeichnet, daß** ein Gemisch aus 0,5-15 Gew-% von einem oder mehreren mehrwertigen Alkoholen mit 3 bis 5 Kohlenstoffatomen, 0,5-10 Gew-% Dihydroxyaceton, 0,05-12 Gew-% organischem alkohollöslichen Sonnenschutzfilter und Wasser unter Rühren für einen Zeitraum von 0,5 bis 1,5 Stunden bei 300 bis 600 U/Min hergestellt wird, und in die erhaltene Lösung 30-85 Gew-% von einem oder mehreren einwertigen Alkoholen mit 2 bis 5 Kohlenstoffatomen und 3-20 Gew-% Parfümöl unter Rühren bis zum Erhalt einer klaren Lösung eingetragen werden.

## Claims

1. A perfume composition which has a tanning effect or both a sun-block and tanning effect comprising in a clear hydroalcoholic solution
an alcohol-soluble, organic sunscreen filter in a proportion of 0.05 to 12 wt. %;
dihydroxyacetone in a proportion of 0.5 to 10 wt. %;
one or more monovalent alcohols with 2 to 5 carbon atoms in a proportion of 30 to 85 wt. %;
one or more polyvalent alcohols with 3 to 5 carbon atoms in a proportion of 0.5 to 15 wt. %;
perfume oil in a proportion of 3 to 20 wt. %; and
water in a proportion of 3 to 67.95 wt. %; and
wherein the proportions refer each time to the total composition.

2. A perfume composition according to claim 1, wherein the concentration of dihydroxyacetone ranges between 3 and 8 wt. %.

3. A perfume composition according to claim 2, wherein the concentration of the perfume oil is in the range of 3 to 15 wt. %.

4. A perfume composition according to claim 1, wherein the concentration of the alcohol-soluble organic sunscreen filter ranges between 1.5 and 10 wt. %.

5. A perfume composition according to claim 1, wherein the organic sunscreen filter is octyl methoxycinnamate.

6. A perfume composition according to claim 1, wherein the proportion of monovalent alcohols ranges between 40 and 80 wt. %.

7. A perfume composition according to claim 1, wherein the monovalent alcohol is ethanol.

8. A perfume composition according to claim 1, wherein the polyvalent alcohol is a mixture of glycerine and 1.3-butane diol.

9. A perfume composition according to any of claims 1 to 8, wherein additionally a water-soluble organic sunscreen filter is contained.

10. A perfume composition according to any of claims 1 to 9, wherein said composition has a sun protection factor between 5 and 10.

11. A perfume composition according to claim 1, wherein said composition is provided as a spray.

12. A method for the production of a perfume composition having a tanning effect or both a sun-block and tanning effect comprising the production of a mixture of 0.5 to 15 wt. % of one or more polyvalent alcohols with 3 to 5 carbon atoms, 0.5 to 10 wt. % of dihydroxyacetone, 0.05 to 12 wt. % of an organic alcohol-soluble sunscreen filter and water by stirring said mixture for a period of 0.5 to 1.5 hours at 300 to 600 rpm and the addition, under stirring, of 30 to 85 wt. % of one or more monovalent alcohols with 2 to 5 carbon atoms and 3 to 20 wt. % of perfume oil until a clear solution is obtained.

## Revendications

1. Composition parfumée ayant un effet autobronzant ou un effet de protection solaire et un effet autobronzant, **caractérisée en ce qu'**elle contient une solution alcoolique - aqueuse transparente, un filtre solaire organique soluble dans l'alcool en une quantité de 0,05 à 12 % en poids, de la dihydroxyacétone en une quantité de 0,5 à 10 % en poids, un ou plusieurs monoalcools avec 2 à 5 atomes de carbone en une quantité de 30 à 85 % en poids, un ou plusieurs polyols avec 3 à 5 atomes de carbone en une quantité de 0,5 à 15 % en poids, une huile parfumée en une quantité de 3 à 20 % en poids et de l'eau en une quantité de 3 à 67,95 % en poids, les quantités étant données en fonction de la composition totale.

2. Composition parfumée selon la revendication 1, **caractérisée en ce que** la concentration de dihydroxyacétone est comprise entre 3 et 8 %.

3. Composition parfumée selon la revendication 2, **caractérisée en ce que** la concentration en huile parfumée est comprise entre 3 et 15 % en poids.

4. Composition parfumée selon la revendication 1, **caractérisée en ce que** la concentration du filtre solaire organique soluble dans l'alcool est comprise entre 1,5 et 10 % en poids.

5. Composition parfumée selon la revendication 1, **caractérisée en ce que** le filtre solaire organique est de l'octyl méthoxycinnamate.

6. Composition parfumée selon la revendication 1, **caractérisée en ce que** la teneur en monoalcool est comprise entre 40 et 80 % en poids.

7. Composition parfumée selon la revendication 1, **caractérisée en ce que** le monoalcool est de l'éthanol.

8. Composition parfumée selon la revendication 1, **caractérisée en ce que** le polyol est un mélange de glycérine et de 1,3-butanediol.

9. Composition parfumée selon l'une des revendications 1 à 8, **caractérisée en ce qu'**en plus un filtre solaire organique hydrosoluble est présent.

10. Composition parfumée selon l'une des revendications 1 à 9, **caractérisée en ce qu'**elle contient un facteur de protection solaire de 5 à 10.

11. Composition parfumée selon la revendication 1, **caractérisée en ce qu'**elle est sous forme de spray.

12. Procédé de fabrication d'une composition parfumée avec un effet autobronzant ou un effet antisolaire et un effet autobronzant, **caractérisé en ce qu'**un mélange de 0,5 à 15 % en poids d'un ou plusieurs monoalcools avec 3 à 5 atomes de carbone, de 0,5 à 10 % en poids de dihydroxyacétone, de 0,05 à 12 % en poids de filtre solaire organique soluble dans l'alcool et d'eau est fabriqué sous agitation pendant 0,5 à 1,5 heure à 300 à 600 U/min et **en ce que**, dans la solution obtenue, 30 à 85 % en poids d'un ou plusieurs monoalcools avec 2 à 5 atomes de carbone et 3 à 20 % en poids d'huile parfumée sont ajoutés sous agitation jusqu'à obtention d'une solution transparente.
